# EUROPEAN PATENT APPLICATION

(11) **EP 3 015 108 A1**
(43) Date of publication of application: **04.05.2016**
(21) Application number: 14817674.6
(22) Date of filing: 27.06.2014
(51) Int. Cl.: A61K 33/00, A61H 33/02, A61K 8/19, A61P 3/00, A61P 9/00, A61P 17/00, A61P 19/00, A61Q 19/10

(54) **CARBONATED WATER-CONTAINING EXTERNAL PREPARATION FOR PROMOTING BLOOD CIRCULATION, BLOOD CIRCULATION-PROMOTING DEVICE FOR SAME, AND BLOOD CIRCULATION-PROMOTING METHOD USING SAME**

(30) Priority: 27.06.2013 JP 2013135453; 29.10.2013 JP 2013224703
(71) Applicant: Mitsubishi Rayon Co., Ltd., Tokyo 100-8253 (JP)
(72) Inventor: TANEIKE, Masahiko, Tokyo 103-0016 (JP); IMAURA, Takashi, Tokyo 103-0016 (JP); KIKUYA, Nobuyuki, Tokyo 103-0016 (JP); ITAKURA, Masanori, Tokyo 100-8253 (JP); INABA, Yoshio, Tokyo 103-0016 (JP); KUMAMOTO, Hideo, Tokyo 100-8251 (JP); NAKAGAWA, Yoshiichi, Tokyo 1030016 (JP); TERAZAWA, Kaoru, Tokyo 103-0016 (JP)
(74) Representative: TBK
(86) International application number: PCT/JP2014/067175
(87) International publication number: WO 2014/208723

(57) **Abstract**

Provided is a blood circulation promoting external preparation containing carbonated water which is applied to a target site of a living body together with ultrasonic irradiation.

## Description

### TECHNICAL FIELD

The present invention relates to a blood circulation promoting external preparation containing carbonated spring, a blood circulation promoting device for the preparation, and a blood circulation promoting method using the preparation.

This application is based upon and claims the benefit of priority of the prior Japanese Patent Application No. 2013-135453 filed in Japan on June 27, 2013 and the prior Japanese Patent Application No. 2013-224703 filed in Japan on October 29, 2013, the entire contents of which are incorporated herein by reference.

### BACKGROUND ART

There are a number of diseases of which the symptom gets worse by poor blood circulation. Examples of the disease having a mild symptom may include excessive sensitivity to cold of lower extremity, muscle fatigue, and shoulder stiffness, and examples of the disease having a severe symptom may include ischemic heart disease, cardiovascular diseases such as cerebral infarction, chronic arterial occlusion, diabetic foot, and venous thrombosis, connective tissue disease-related diseases such as chronic articular rheumatism or Raynaud's syndrome, and skin diseases including intractable ulcer such as bedsore.

In these diseases, the supply of nutrients and oxygen to the tissue decreases and the tissue is damaged when the blood vessel is contracted and the circulation of blood is stagnated. In addition, the regeneration and healing ability of the tissue decreases when the impeded blood flow is not improved so that the tissue damage advances and the condition is getting worse.

Hence, not only worsening of the symptom is suppressed but also an effect to promote healing of the disease is expected when the blood circulation in the whole body or affected area of the patients who suffer from these diseases is promoted.

Meanwhile, a medicated bath of a carbonated spring had been used in the medical treatment of diseases in Europe since ancient times.

On the other hand, in Japan, there are a small number of high-concentration carbonated springs and also a number of the high-concentration carbonated springs are a mineral spring and have a low water temperature, and thus the carbonated spring has not attracted attention in Japan where a hot spring is strongly preferred.

In recent years, the medicated bath of a high-concentration carbonated spring has been clinically reviewed as it has an effect to promote blood circulation. Moreover, it is said that the effect of the medicated bath of a carbonated spring is higher as the concentration of carbonic acid dissolved therein is higher.

Examples of the method for producing high-concentration carbonated water, that is, the method to artificially dissolve carbonic acid in water may include a chemical method in which a tablet or the like containing sodium bicarbonate is introduced into water, a method in which carbonic acid is mixed with water and dissolved by applying pressure, a method using a static mixer, and a method using a multi-layer composite hollow fiber membrane.

Among these methods, the method using a multi-layer composite hollow fiber membrane is suitable for mass production of the medicated bath solution of high-concentration carbonated water (Patent Document 1).

The clinical efficacy of the medicated bath of high-concentration carbonated water produced using a multi-layer composite hollow fiber membrane has been demonstrated so far.

For example, bathing in high-concentration carbonated water produced using a multi-layer composite hollow fiber membrane has a physiological action such as skin flushing, suppression of skin cold sensation, a decrease in core temperature, a decrease in heart rate, an increase in skin blood flow is indicated in Non-Patent Document 1. In addition, the fact that the medicated bath of high-concentration carbonated water has a therapeutic effect against articular rheumatism and the quality of life (QOL) of patients is improved is also indicated in the same document.

In addition, the fact that an action to promote the healing of bedsore has been obtained as a patient with bedsore takes the medicated bath of high-concentration carbonated water is indicated in Non-Patent Document 2.

### CITATION LIST

### PATENT DOCUMENT

Patent Document 1: JP 2009-285651 A

### NON-PATENT DOCUMENT

Non-Patent Document 1: IRIKI et al., "Physiological effect of artificial carbonated bath", carbonated spring magazine, 1999, Vol. 2, No. 2, p. 30 to 35
Non-Patent Document 2: KABURAGI et al., "Study on healing effect of full-body bathing in high-concentration artificial carbonated spring against bedsore", carbonated spring magazine, 2000, Vol. 3, No. 1, p. 15 to 20

### SUMMARY OF THE INVENTION

### PROBLEM TO BE SOLVED BY THE INVENTION

However, it cannot be said that the therapeutic effect in Non-Patent Documents 1 and 2 is sufficiently satisfactory, and thus the development of use of the medicated bath of high-concentration carbonated water exhibiting a higher blood circulation promoting effect is desired.

Accordingly, an object of the invention is to provide a blood circulation promoting external preparation containing carbonated water having a higher blood circulation promoting effect, a blood circulation promoting device for the preparation, and a blood circulation promoting method using the preparation.

### MEANS FOR SOLVING PROBLEM

As a result of intensive investigations, the present inventors have found out a new use of the medicated bath of high-concentration carbonated water having a higher blood circulation promoting effect than Non-Patent Documents 1 and 2. More specifically, the present inventors have found out that the blood circulation promoting effect is remarkably improved by concurrently using the blood circulation promoting external preparation containing carbonated water with ultrasonic irradiation. Although it has been known that ultrasonic irradiation itself also has a blood circulation promoting effect, a sufficient therapeutic effect has not been obtained. However, it has been found out that the blood circulation can be significantly improved by a synergistic effect exceeding the expected extent due to concurrent use of carbonated water and ultrasonic irradiation, whereby the invention has been completed.

In other words, the invention includes the following configurations.
[1] A blood circulation promoting external preparation containing carbonated water, in which the blood circulation promoting external preparation is applied to a target site of a living body together with ultrasonic irradiation.
[2] The blood circulation promoting external preparation described in [1], in which a concentration of carbonic acid dissolved in the blood circulation promoting external preparation is 100 ppm or more.
[3] The blood circulation promoting external preparation described in [1] or [2], in which the application of the blood circulation promoting external preparation to a target site is carried out by immersion of a target site in the blood circulation promoting external preparation, coating of the blood circulation promoting external preparation on a target site, or continuous or intermittent supply of the blood circulation promoting external preparation to a target site.
[4] The blood circulation promoting external preparation described in any one of [1] to [3], in which the ultrasonic irradiation has a step of generating a vibratory pressure in an ultrasonic region in a blood circulation promoting external preparation or with respect to a target site.
[5] The blood circulation promoting external preparation described in any one of [1] to [4], in which the carbonated water is prepared at the time of use.
[6] The blood circulation promoting external preparation described in any one of [1] to [5], in which the blood circulation promoting external preparation is kept warm at from 30 to 50°C at the time of use.
[7] The blood circulation promoting external preparation described in any one of [1] to [6], in which the number of vibrations in an ultrasonic region in the ultrasonic irradiation is from 16 kHz to 10 MHz.
[8] The blood circulation promoting external preparation described in any one of [1] to [7], in which the blood circulation promoting external preparation is furhter used in a state in which a vortex is generated.
[9] The blood circulation promoting external preparation described in any one of [1] to [8], in which the blood circulation promoting external preparation is for treatment or prevention of a disease, treatment of an external injury, or beauty.
[10] The blood circulation promoting external preparation described in [9], in which the disease is selected from the group consisting of a cardiovascular disease, a bone disease, a skin disease, and an infectious ulcer.
[11] A blood circulation promoting device including: an external preparation applying mechanism to apply a blood circulation promoting external preparation containing carbonated water to a target site; and an ultrasound irradiating mechanism to generate a vibratory pressure in an ultrasonic region and to irradiate the target site with the vibratory pressure.
[12] The blood circulation promoting device described in [11], in which a concentration of carbonic acid dissolved in the blood circulation promoting external preparation is 100 ppm or more.
[13] The blood circulation promoting device described in [11] or [12], in which the external preparation applying mechanism is constituted so that the application of the blood circulation promoting external preparation to a target site is carried out by immersion of a target site in the blood circulation promoting external preparation, coating of the blood circulation promoting external preparation on a target site, or continuous or intermittent supply of the blood circulation promoting external preparation to a target site.
[14] The blood circulation promoting device described in any one of [11] to [13], in which the ultrasound irradiating mechanism has a device to generate a vibratory pressure in an ultrasonic region in a blood circulation promoting external preparation or with respect to a target site.
[15] The blood circulation promoting device described in any one of [11] to [14], further including a mechanism to dissolve carbonic acid in water.
[16] The blood circulation promoting device described in any one of [11] to [15], further including a mechanism to control a temperature of the blood circulation promoting external preparation.
[17] The blood circulation promoting device described in any one of [11] to [16], further including a mechanism to generate a vortex in the blood circulation promoting external preparation.
[18] The blood circulation promoting device described in any one of [11] to [17], in which the blood circulation promoting device is for treatment or prevention of a disease, treatment of an external injury, or beauty.
[19] The blood circulation promoting device described in [18], in which the disease is selected from the group consisting of a cardiovascular disease, a bone disease, a skin disease, and an infectious ulcer.
[20] A blood circulation promoting method including a step of applying the blood circulation promoting external preparation described in any one of [1] to [10] to a target site as well as generating a vibratory pressure in an ultrasonic region and irradiating the target site with the vibratory pressure.
[21] The blood circulation promoting method described in [20], in which the blood circulation promoting method is for treatment or prevention of a disease, treatment of an external injury, or beauty.
[22] The blood circulation promoting method described in [21], in which the disease is selected from the group consisting of a cardiovascular disease, a bone disease, a skin disease, and an infectious ulcer.
[23] The blood circulation promoting method described in any one of [20] to [22], in which the blood circulation promoting external preparation is applied to the target site at a temperature of from 30 to 50°C.

### EFFECT OF THE INVENTION

According to the blood circulation promoting external preparation, blood circulation promoting device, or blood circulation promoting method of the invention, it is possible to obtain a blood circulation promoting effect which is remarkably improved compared to that of the prior art.

### BRIEF DESCRIPTION OF DRAWINGS

Fig. 1 is a schematic diagram of a blood circulation promoting device according to an embodiment of the invention;
Fig. 2 is a schematic diagram of the blood circulation promoting device according to another embodiment of the invention;
Fig. 3 is a graph illustrating the evaluation results of the blood circulation promoting action in a test subject according to Examples using the device illustrated in Fig. 1;
Fig. 4 is a graph illustrating the evaluation results of the blood circulation promoting action in another test subject according to Examples using the device illustrated in Fig. 1;
Fig. 5 is a graph illustrating the evaluation results of the blood circulation promoting action depending on a difference in ultrasonic vibratory pressure (sound pressure) level;
Fig. 6 is a graph illustrating the evaluation results of the blood circulation promoting action depending on a difference in carbonic acid concentration level in carbonated water;
Fig. 7 is a graph illustrating the evaluation results of the blood circulation promoting action in a test subject according to Example using the device illustrated in Fig. 2;
Fig. 8 is a graph illustrating the evaluation results of the blood circulation promoting action in another test subject according to Example using the device illustrated in Fig. 2; and
Fig. 9 is a graph illustrating the evaluation results of the blood circulation promoting action depending on a difference in distance to a test subject at the time of ultrasonic irradiation.

### MODE(S) FOR CARRYING OUT THE INVENTION

Hereinafter, the invention will be described in detail.

### <Blood circulation promoting external preparation>

The blood circulation promoting external preparation of the invention contains carbonated water and is characterized by being concurrently used with ultrasonic irradiation. In other words, according to the invention, the use of carbonated water as a blood circulation promoting external preparation which is applied to the target site of a living body together with ultrasonic irradiation is provided.

Furthermore, the temperature of the blood circulation promoting external preparation of the invention may be set to from 30 to 50°C or a vortex may be generated therein at the time of use.

The blood circulation promoting external preparation of the invention is in a solution form and a gel form. Examples of the main solvent may include fresh water, but it is not limited thereto.

In addition, the blood circulation promoting external preparation of the invention may contain an antibiotic, a vitamin, a sterilized physiological saline, and the like.

### (Carbonated water)

Carbonated water in the invention means water having a higher concentration of carbonic acid dissolved therein compared to fresh water, and it refers to water in which carbonic acid is dissolved at 20 ppm or more.

It is desirable that the concentration of carbonic acid dissolved in carbonated water is at least 100 ppm in order to obtain high efficacy by carbonated water.

Incidentally, the setting of the concentration of carbonic acid dissolved in the blood circulation promoting external preparation containing carbonated water is more important than the setting of the concentration of carbonic acid dissolved in carbonated water in order to obtain sufficient drug efficacy according to the invention.

Hence, the concentration of carbonic acid dissolved in the blood circulation promoting external preparation of the invention is preferably 100 ppm or more, more preferably 250 ppm or more, even more preferably 500 ppm or more, particularly 700 ppm or more, and extremely preferably 1000 ppm or more. Incidentally, the concentration of carbonic acid dissolved can be measured by a known method, for example, a titration method.

As carbonated water of the invention, those having carbonic acid already dissolved therein at a desired concentration may be used or carbonated water may be prepared at the time of use. It is relatively preferable to prepare carbonated water at the time of use when it is considered that carbonic acid is time-dependently released from the blood circulation promoting external preparation.

The term "preparation at the time of use" in the present specification means that carbonic acid is artificially dissolved in water at the time of use or before use of the blood circulation promoting external preparation.

In addition, the term "before use" in the present specification means sufficiently before the concentration of carbonic acid is increased in advance so as to obtain the effect of the invention at the time of use of the blood circulation promoting external preparation. For example, it is preferably within 24 hours before the start of use and more preferably within 10 minutes before the start of use.

In addition, it is also possible to monitor the concentration of carbonic acid using a carbonic acid gas concentration measuring instrument or the like during use of the blood circulation promoting external preparation and to additionally dissolve carbonic acid in the preparation in a case in which the concentration of carbonic acid is equal to or less than a desired concentration.

Examples of the method for artificially dissolving carbonic acid in water may include a chemical method in which a tablet or the like containing sodium bicarbonate is introduced into water, a method in which carbonic acid is mixed with water and then dissolved by applying pressure, a method using a static mixer, a method using a multi-layer composite hollow fiber membrane, and a method in which carbonic acid is dissolved by breaking the bubbles thereof into fine bubbles.

Among these methods, a method using a static mixer or a method using a multi-layer composite hollow fiber membrane is preferable from the viewpoint of maintaining carbonic acid at a high concentration as well as the dissolution efficiency and the miniaturization of device. In both methods, the concentration of carbonic acid may be increased by allowing water to pass through a static mixer or a multi-layer composite hollow fiber membrane plural times or the concentration of carbonic acid may be increased by allowing water to pass through them one time (in one pass). In particular, a method utilizing a multi-layer composite hollow fiber membrane is suitable for mass production or preparation at the time of use since high-concentration carbonated water is obtained even in one pass.

### (Vibratory pressure in ultrasonic region)

The blood circulation promoting external preparation of the invention is applied while irradiating the target site with a vibratory pressure in the ultrasonic region at the time of use. The method for generating a vibratory pressure in the ultrasonic region at that time may be a step of generating a vibratory pressure in the ultrasonic region in the blood circulation promoting external preparation or a step of generating a vibratory pressure in the ultrasonic region with respect to the target site.

The term "ultrasonic region" in the blood circulation promoting external preparation of the invention is defined as those which the human cannot hear among the oscillatory waves generated in the blood circulation promoting external preparation. In general, the upper limit of the number of vibrations which the human can hear is about 16 kHz. Hence, the example of the "ultrasonic region" in the invention refers to a high-frequency band of 16 kHz or more.

The number of vibrations of an ultrasound in the ultrasonic irradiation of the invention is preferably from 16 kHz to 10 MHz, more preferably from 100 kHz to 5 MHz, and even more preferably from 1 MHz to 3 MHz. A higher blood circulation promoting effect is obtained when the frequency is equal to or more than the lower limit value and the load on a living body is small when the frequency is equal to or less than the upper limit value.

As the method for generating a vibratory pressure in the ultrasonic region, the vibratory pressure may be generated by using an ultrasonic generator that is generally used.

Examples of the method for generating a vibratory pressure in the ultrasonic region in the case of generating it in the blood circulation promoting external preparation may include a method using an ultrasound generating nozzle as disclosed in JP 2008-206679 A in addition to a method in which various kinds of ultrasonic transducers are dipped in the blood circulation promoting external preparation to generate a vibratory pressure in the ultrasonic region.

In the case of using an ultrasound generating nozzle, it is also possible to provide the ultrasound generating nozzle with a suction pipe, to suck air by the Venturi effect, and to mix the air with carbonated water so as to generate small bubbles.

The vibratory pressure in the ultrasonic region generated in the blood circulation promoting external preparation has the hyperthermic action or the micro-massage action so that the therapeutic effect of the invention is enhanced.

Examples of the method for generating a vibratory pressure in the ultrasonic region in the case of generating the vibratory pressure in the atmosphere may include a method using an ultrasonic therapeutic system described in JP 2007-521053 W.

In addition, the sound energy received by the target site from the position from which the ultrasound is irradiated in the case of coating is preferably from 15 dB to 110 dB and more preferably from 20 dB to 110 dB.

### (Temperature at time of use)

A sufficient drug efficacy is obtained from the blood circulation promoting external preparation of the invention even at room temperature, but the preparation may be heated and used in order to further enhance the drug efficacy.

The temperature is preferably from 30 to 50°C and more preferably from 35 to 45°C. A higher blood circulation promoting effect is obtained when the temperature is equal to or more than the lower limit value, and the blood circulation promoting external preparation can be applied for a long period of time when the temperature is equal to or less than the upper limit value.

Thermal insulation is conducted by using a heater for thermal insulation which is generally used for the thermal insulation of water or a container. It is preferable to keep warm the blood circulation promoting external preparation in order to use it within the above temperature range. The kind of the heater for thermal insulation is not particularly limited as long as it can be used on the body, and the heater may be for home use or industrial use.

Examples of the method for using a heater for thermal insulation may include a method in which carbonated water which is already adjusted to a desired temperature is introduced into a bathtub at the time of starting application of the blood circulation promoting external preparation and the heater for thermal insulation is used for thermal insulation of the blood circulation promoting external preparation during the application. Alternatively, examples thereof may also include a method in which carbonated water which has not reached a predetermined temperature is heated by a heater for thermal insulation and the heater for thermal insulation is also used for thermal insulation of the blood circulation promoting external preparation during the application.

### (Vortex)

A vortex may be generated in the case of using the blood circulation promoting external preparation of the invention as a bath.

The vortex is generated so that the pressure is applied to the whole body or a local part.

The pressure of the vortex (the discharge pressure from the mechanism to generate the vortex) is preferably from 10 to 320 kPa and more preferably from 50 to 200 kPa. A higher blood circulation promoting effect is obtained when the pressure of the vortex is equal to or more than the lower limit value and a problem that a water wing or the ability of device becomes excessive or the like is not caused when the pressure of the vortex is equal to or less than the upper limit value.

As the method for generating a vortex, the vortex is generated by using a water flow generator which is generally used.

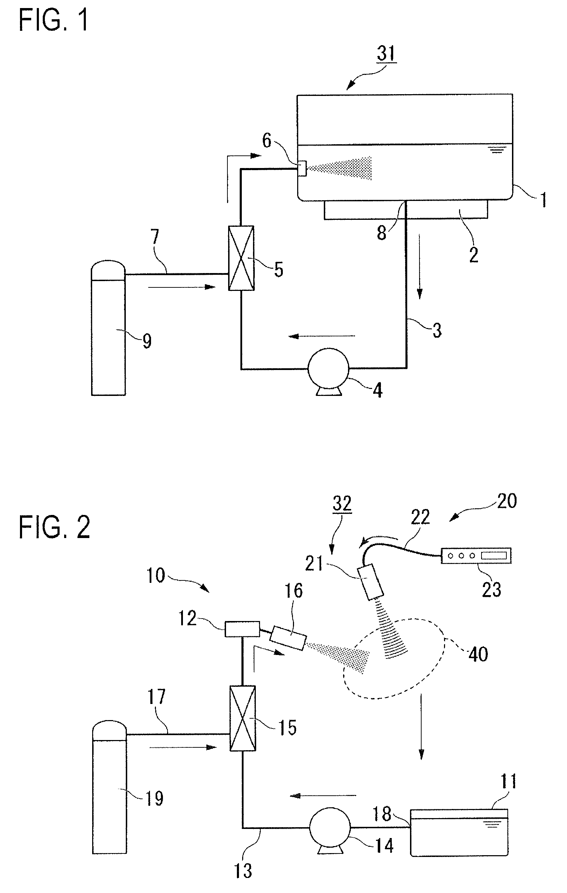

Examples thereof may include a method in which a water inlet 8 and a discharge nozzle 6 are provided on the wall or bottom of a bathtub 1, a circulation pipe 3 to circulate carbonated water is provided from the water inlet 8 to the discharge nozzle 6, and a pump 4 is provided in the middle of the circulation pipe 3 as illustrated in Fig. 1. In this method, a vortex is generated when the blood circulation promoting external preparation is circulated from the bathtub 1 to the circulation pipe 3 and returns to the bathtub 1 again through the discharge nozzle 6. The vortex causes a moderate pressure to the whole body or a local part which is immersed in the blood circulation promoting external preparation stored in the bathtub 1.

A massage effect is obtained in the body part to which the pressure is applied by the vortex, and thus the blood flow can be further promoted.

### (Arbitrary component)

The blood circulation promoting external preparation of the invention may contain a component having a drug efficacy, an additive, and the like other than carbonic acid.

Examples of the component having a drug efficacy may include an antibiotic, an analgesic, and an antiphlogistic.

Examples of the additive may include a perfume, an algefacient, and a preservative which are generally used as the additives of an external preparation. In addition, in a case in which the applied site includes mucosa, a salt such as sodium chloride and potassium chloride may be added to the blood circulation promoting external preparation in order to prepare an isotonic solution or a pH adjusting agent such as phosphate buffer may be contained in the blood circulation promoting external preparation in order to suppress the irritation to the mucosa.

### (Applied place)

The blood circulation promoting external preparation of the invention is applied to the whole body or a local part of an animal, particularly, the mammal such as human.

The whole body in the invention means the whole body in a range in which breathing can be secured. For example, in the case of the mammal, it usually means that the body except the head, namely, the neck and the lower part of the body are dipped in the blood circulation promoting external preparation.

The local part in the invention is not particularly limited as long as it is a part of the body, and examples thereof may include head, face, neck, shoulders, arms, hands, chest, abdomen, buttocks, and legs. The blood circulation promoting external preparation of the invention may be applied to plural parts of the body at the same time.

### (Applying method)

Examples of the method for applying the blood circulation promoting external preparation of the invention may include a method in which the preparation is applied by immersing the target site in carbonated water, a method in which the preparation is applied by coating carbonated water on the target site through spraying, jetting out, or the like, and a method in which the preparation is applied by continuously or intermittently supplying carbonated water to the target site through showering or pouring of the carbonated water onto the target site from the container so as to flow thereon.

In addition, the applied amount of the blood circulation promoting external preparation is not particularly limited as long as it is the amount by which the blood circulation promoting effect is obtained. For example, in the case of a method to immerse, it depends on the size of the container used in order to hold the blood circulation promoting external preparation, but it may be an amount that is enough to immerse the target site. In the case of a method to coat, for example, the amount per area of the target site is preferably from 1 ml to 15 L/cm², and a more preferable applied amount and the supplying method can be appropriately selected in consideration of the size, state, and the like of the target site.

### (Applied subject)

The subject to which the blood circulation promoting external preparation of the invention is applied is not particularly limited as long as the subject requires the blood circulation promoting action.

For example, the preparation may be for treatment or prevention of a disease, treatment of an external injury, or beauty, furthermore, it may be used for obtaining the fatigue recovery effect on shoulder stiffness, muscle pain, or the like or a detox effect.

In the case of using the preparation in the treatment or prevention of a disease, the disease to be the subject is a disease which desires the blood circulation promoting action, that is, it is more preferably a disease of which the symptom gets worse by poor blood circulation. Examples of the disease of which the symptom gets worse by poor blood circulation may include oversensitiveness to cold of lower extremity, muscle fatigue, ischemic heart disease, cardiovascular diseases such as cerebral infarction, clu-onic arterial occlusion, diabetic foot, and venous thrombosis, connective tissue disease-related diseases such as chronic articular rheumatism or Raynaud's syndrome, skin diseases including intractable ulcer such as bedsore, and infectious ulcers (ulcers caused by pathogenic bacteria) such as a tropical ulcer. It is possible to suppress worsening of the symptom or to improve the symptom when the blood circulation promoting external preparation of the invention is applied to a subject suffering from these diseases.

In the case of using the preparation in the treatment of an external injury, examples of the external injury to be the subject may include a gunshot wound and an injury caused by a traffic accident. In this case, the blood circulation promoting external preparation of the invention is mainly effective for alleviating and treating various symptoms due to the hematogenous disorder seen after healing of bleeding, a lacerated wound, or the like.

In addition, the skin is shiny by the blood circulation promoting action in the skin when the blood circulation promoting external preparation of the invention is used for beauty.

When the preparation is used for the treatment of shoulder stiffness, muscle pain, or the like, it is possible to recover the stiffness caused by vasoconstriction in the muscle and also to remove the lactic acid accumulated in the muscle by the blood circulation promoting effect of the invention.

### (Applied time)

It is preferable to immerse the applied place in the blood circulation promoting external preparation for from 10 to 60 minutes in order to obtain the effect by the blood circulation promoting external preparation of the invention, and the applied time is more preferably from 20 to 40 minutes. A higher blood circulation promoting effect is obtained when the applied time is equal to or more than the lower limit value, and the burden of maintaining the posture is small when the applied time is equal to or less than the upper limit value.

### (Applied frequency)

The applied frequency of the blood circulation promoting external preparation of the invention may be appropriately determined based on the improvement in symptom and the like, but it is possible to obtain a desired effect by, for example, applying the preparation about from one time to several times per one day over a period of about from 3 days to 180 days.

### <Blood circulation promoting device>

The blood circulation promoting device of the invention has an external preparation applying mechanism to apply a blood circulation promoting external preparation containing carbonated water to a target site and an ultrasound irradiating mechanism to generate a vibratory pressure in the ultrasonic region and to irradiate the target site with the vibratory pressure.

The blood circulation promotion promoting device of the invention may further have a "mechanism to dissolve carbonic acid in water", a "mechanism to heat or keep warm the blood circulation promoting external preparation", and a "mechanism to generate a vortex".

A schematic diagram of an embodiment of the blood circulation promoting device of the invention is illustrated in Fig. 1. However, the disposition of the respective mechanisms in the present embodiment is not limited thereto.

The bathtub 1 is provided with the water inlet 8 and the discharge nozzle 6. The circulation pipe 3 to circulate water or the blood circulation promoting external preparation is provided between the water inlet 8 and the discharge nozzle 6. In the middle of this circulation pipe 3, the pump 4 and a gas dissolving unit 5 are provided. Water or the blood circulation promoting external preparation stored in the bathtub 1 is absorbed through the water inlet 8 by the pump 4, passes through the circulation pipe 3 and the gas dissolving unit 5, and returns to the bathtub 1 again through the discharge nozzle 6.

Between the water inlet 8 and the discharge nozzles 6, a heating unit 2, the pump 4, and the gas dissolving unit 5 are disposed in order. In addition, a bomb 9 is connected to the gas dissolving unit 5 via a carbonic acid gas pipe 7 so that carbonic acid gas is sent from the bomb 9 into the gas dissolving unit 5 via the carbonic acid gas pipe 7.

In the case of using a blood circulation promoting device 31 in Fig. 1, the amount of the blood circulation promoting external preparation circulated is preferably from 5 to 20 L/min and more preferably from 8 to 15 L/min. The effect by a vibratory pressure in the ultrasonic region is sufficiently obtained when the amount of the preparation circulated is equal to or more than the lower limit value, and the blood circulation promoting external preparation does not spring up from the water surface when the amount of the preparation circulated is equal to or less than the upper limit value.

Incidentally, in Fig. 1, the heating unit 2 corresponds to the "mechanism to heat or keep warm the blood circulation promoting external preparation", and the gas dissolving unit 5 corresponds to the "mechanism to dissolve carbonic acid in water". In addition, the discharge nozzle 6 has both mechanisms of the "mechanism to generate a vibratory pressure in the ultrasonic region in the blood circulation promoting external preparation containing carbonated water" and the "mechanism to generate a vortex". Hereinafter, the respective configurations of the blood circulation promoting device 31 of the present embodiment will be described in detail.

### (Carbonated water)

The definition of carbonated water to be used in the blood circulation promoting device 31 of the present embodiment is the same as that described in the section of the "blood circulation promoting external preparation".

### (Mechanism to generate vibratory pressure in ultrasonic region and to irradiate target region with vibratory pressure)

In the mechanism to generate a vibratory pressure in the ultrasonic region and to irradiate the target region with the vibratory pressure, the vibratory pressure in the ultrasonic region can be generated by a device to generate a vibratory pressure in the ultrasonic region in the blood circulation promoting external preparation.

Incidentally, the definition of the "vibratory pressure in the ultrasonic region" and the action by the vibratory pressure are the same as those described in the section of the "blood circulation promoting external preparation".

"Device to generate vibratory pressure in ultrasonic region in blood circulation promoting external preparation"

In the mechanism to generate a vibratory pressure in the ultrasonic region and to irradiate the target region with the vibratory pressure of the present embodiment, a vibratory pressure in the ultrasonic region is emitted towards the blood circulation promoting external preparation stored in the bathtub 1 and the whole body or a local part dipped in the blood circulation promoting external preparation is irradiated with the vibratory pressure.

Examples of the device may include a general underwater ultrasonic generator that can be applied to the body. Specific examples thereof may include an ultrasound generating nozzle which can enhance the vibratory pressure level to a high frequency band of 16 kHz or more and is disclosed in JP 2008-206679 A in addition to a combination of an ultrasonic source and an ultrasonic transducer.

In the case of using an ultrasound generating nozzle, the mechanism may be provided instead of the discharge nozzle 6 in Fig. 1.

Incidentally, the definition of the "vibratory pressure in the ultrasonic region" and the action by the vibratory pressure are the same as those described in the section of the "blood circulation promoting external preparation".

### Bathtub 1:

In the mechanism to generate a vibratory pressure in the ultrasonic region and to irradiate the target region with the vibratory pressure, the bathtub 1 for dipping the whole body or a local part in the blood circulation promoting external preparation is required in the case of using a device to generate a vibratory pressure in the ultrasonic region in the blood circulation promoting external preparation.

In the present embodiment, the bathtub 1 is a container storing carbonated water in order to dip the whole body or a local part in carbonated water and to irradiate the whole body or the local part with a vibratory pressure in the ultrasonic region.

As the bathtub 1, arbitrary one that is different from the blood circulation promoting device 31 may be used, or the bathtub 1 may be provided so as to be integrated with the blood circulation promoting device 31.

In the case of providing the bathtub 1 so as to be integrated with the blood circulation promoting device 31, the "mechanism to generate a vibratory pressure in the ultrasonic region in the blood circulation promoting external preparation containing carbonated water" described above is provided to the wall or bottom of the bathtub 1 so that the vibratory pressure is generated in the blood circulation promoting external preparation in the bathtub 1.

### (Mechanism to heat or keep warm blood circulation promoting external preparation)

A sufficient effect is obtained even without thermal insulation, but the blood circulation promoting device 31 of the present embodiment may have the "mechanism to heat or keep warm the blood circulation promoting external preparation" in order to further enhance the effect.

As the mechanism, for example, the "heater for thermal insulation" described in the section of the "blood circulation promoting external preparation" is used.

The mechanism may be provided, for example, in the middle of the circulation pipe 3 as illustrated in Fig. 1 or may be provided in the middle of a pipe which is provided with the pump separately from the circulation pipe 3. Alternatively, the mechanism may be provided so as to be in contact with the outer side of the wall or bottom of the bathtub 1 and to heat or keep warm water or the blood circulation promoting external preparation in the bathtub 1 by thermally conducting the wall or bottom of the bathtub 1, or the mechanism may be provided so as to be dipped in water or the blood circulation promoting external preparation in the bathtub 1 and to directly heat or keep warm water or the preparation.

With regard to the temperature range, a higher blood circulation promoting effect is obtained when the temperature is set to 30°C or higher, and it is possible to apply the blood circulation promoting external preparation for a long period of time when the temperature is set to 50°C or lower. In addition, it is possible to expect a higher effect when the temperature range is set to from 35 to 45°C instead of from 30 to 50°C.

In addition, the mechanism may have a thermostat in order to maintain these temperature ranges.

### (Mechanism to dissolve carbonic acid in water)

As carbonated water to be used in the blood circulation promoting device 31 of the present embodiment, a commercially available product may be used or carbonated water may be prepared at the time of use by the "mechanism to dissolve carbonic acid in water".

Examples of the "mechanism to dissolve carbonic acid in water" may include a chemical method in which a tablet or the like containing sodium bicarbonate is introduced into water, a method in which carbonic acid is mixed with water and then dissolved by applying pressure, a method using a static mixer, and a method using a multi-layer composite hollow fiber membrane.

Among these methods, a method using a static mixer or a method using a multi-layer composite hollow fiber membrane is preferable from the viewpoint of the dissolution efficiency and the miniaturization of device.

The mechanism may be provided in the middle of the circulation pipe 3 as illustrated in Fig. 1 or may be provided in the middle of a pipe which is provided with the pump separately from the circulation pipe 3.

### (Mechanism to generate vortex)

The blood circulation promoting device 31 of the present embodiment may be provided with a "mechanism to generate a vortex".

The "mechanism to generate a vortex" in the present embodiment is a mechanism to jet out carbonated water to carbonated water in the bathtub 1 and to apply the pressure by the vortex to the whole body or a local part dipped in the blood circulation promoting external preparation containing carbonated water.

Hence, the mechanism is provided to a place inside the bathtub 1 at which the pressure by the vortex can be applied to the whole body or a local part dipped in the blood circulation promoting external preparation containing carbonated water.

In addition, the mechanism may be provided to the discharge nozzle 6 in Fig. 1 or may be provided to a discharge nozzle which is provided with a pipe and a pump separately from the discharge nozzle 6.

In the case of providing the mechanism to the discharge nozzles 6 in Fig. 1, the "mechanism to generate a vibratory pressure in the ultrasonic region in the blood circulation promoting external preparation containing carbonated water" may be continuously provided to the discharge nozzle 6.

Incidentally, the effect of the vortex is the same as those described in the section of the "blood circulation promoting external preparation".

### (Target site)

The blood circulation promoting device 31 of the present embodiment is applied to the whole body or a local part in the same manner as in the section of the "blood circulation promoting external preparation" described above. In addition, the definition of this "whole body" or "local part" is also the same as that in the section of the "blood circulation promoting external preparation" described above.

### (Applying method)

As the applying method when using the blood circulation promoting device 31 of the present embodiment, a target site is immersed in the blood circulation promoting external preparation stored in the bathtub 1 described above.

### (Applied subject)

The subject to which the blood circulation promoting device 31 of the present embodiment is applied is not particularly limited as long as the subject requires the blood circulation promoting action in the same manner as in the section of the "blood circulation promoting external preparation" described above. Examples of the applied subject are the same as those in the section of the "blood circulation promoting external preparation" described above.

### (Applied time)

It is preferable to apply a target site for from 10 to 60 minutes in order to obtain the effect by the blood circulation promoting device 31 of the present embodiment, and the applied time is more preferably from 20 to 40 minutes. A higher blood circulation promoting effect is obtained when the applied time is equal to or more than the lower limit value, and the burden of maintaining the posture is small when the applied time is equal to or less than the upper limit value.

### (Another embodiment)

A schematic diagram of another embodiment of the blood circulation promoting device of the invention is illustrated in Fig. 2. However, the disposition of the respective mechanisms in the present embodiment is not limited thereto.

A blood circulation promoting device 32 of the present embodiment has a carbonated water producing unit 10 and an ultrasonic vibratory pressure generating unit 20.

In the carbonated water producing unit 10, a container 11 is provided with a water inlet 18 and filled with a solvent for carbonated water production, such as water at the time of use. A pipe 13 for sending water is provided between the water inlet 18 and a spray or shower head 16. In the middle of this pipe 13, a pump 14, a gas dissolving unit 15, and a heating unit 12 are provided. The solvent, such as water, stored in the container 11 is absorbed through the water outlet 18 by the pump 14, passes through the pipe 13 and the gas dissolving unit 15, and is sprayed through the spray head or is jetted out through the shower head so as to be applied to a target site 40.

Between the water inlet 18 and the spray or shower head 16, the heating unit 12, the pump 14, and the gas dissolving unit 15 are disposed in order. In addition, a bomb 19 is connected to the gas dissolving unit 15 via a carbonic acid gas pipe 17 so that carbonic acid gas is sent from the bomb 19 into the gas dissolving unit 15 via the carbonic acid gas pipe 17.

The ultrasonic vibratory pressure generating unit 20 has a transducer 21, a cord 22, and a body 23 of the ultrasonic vibratory pressure generator.

A vibratory pressure in the ultrasonic region is generated in the atmosphere from the transducer 21, and the target site 40 is irradiated with the vibratory pressure by adjusting the direction of the transducer 21. In addition, the strength of the vibratory pressure in the ultrasonic region is controlled by a power source 23 via the cord 22.

Incidentally, in the present embodiment, the carbonated water producing unit 10 corresponds to the external preparation applying mechanism, and the ultrasonic vibratory pressure generating unit 20 corresponds to the ultrasound irradiating mechanism.

In addition, the heating unit 12 corresponds to the "mechanism to heat or keep warm the blood circulation promoting external preparation", and the gas dissolving unit 15 corresponds to the "mechanism to dissolve carbonic acid in water". In other words, in the present embodiment, the carbonated water producing unit 10 has these mechanisms.

Hereinafter, the respective configurations of the blood circulation promoting device 32 of the present embodiment will be described in detail.

The definition of carbonated water to be used in the blood circulation promoting device 32 of the present embodiment is the same as that described in the section of the "blood circulation promoting external preparation".

In addition, the mechanism to dissolve carbonic acid in water in the present embodiment is the same as that in the embodiment of Fig. 1 described above.

Examples of the ultrasonic vibratory pressure generating unit 20 of the present embodiment may include the US-750 manufactured by Ito Co., Ltd..

Incidentally, the definition of the "vibratory pressure in the ultrasonic region" and the action by the vibratory pressure are the same as those described in the section of the "blood circulation promoting external preparation".

The mechanism to heat or keep warm the blood circulation promoting external preparation of the present embodiment is basically the same as that in the embodiment of Fig. 1 described above, but there are the following things to keep in mind with regard to the installation place and the temperature setting.

The mechanism may be provided, for example, in the middle of the pipe 13 as illustrated in Fig. 2 or may be provided to the container 11. In order to adjust the temperature at the target site 40, the mechanism is provided preferably in the middle of the pipe 13 and more preferably at a place close to the spray or shower head 16 even in the middle of the pipe 13.

In the present embodiment, the mechanism is adjusted so as to have a desired temperature at the target site 40.

The applying method when using the blood circulation promoting device 32 of the present embodiment, the blood circulation promoting external preparation is applied by being sprayed or jetted out towards a target site through the spray or shower head 16. In this case, the supply of the blood circulation promoting external preparation may be stopped at the time point at which the target site is sufficiently covered with the blood circulation promoting external preparation by coating the blood circulation promoting external preparation on the target site and the target site may be irradiated with ultrasound, but it is more preferable to continuously or intermittently supply the blood circulation promoting external preparation to the target site in order to obtain a greater blood circulation promoting effect.

As the spray head and the shower head, those which are used at the bathtubs, the washbasins, and the like can be used. In addition, the blood circulation promoting external preparation may be applied by pouring it onto the target site from the container so as to flow thereon instead of using a spray head or a shower head.

Incidentally, the target site, the applied subject, the applied time, and the applied frequency in the present embodiment are the same as those in the embodiment of Fig. 1 described above.

### <Blood circulation promoting method>

The blood circulation promoting method of the invention includes a step of applying the blood circulation promoting external preparation described above to a target site as well as generating a vibratory pressure in the ultrasonic region and irradiating the target site with the vibratory pressure.

Carbonated water, the vibratory pressure in the ultrasonic region, the thermal insulation, the vortex, an arbitrary component, the target site, the applying method, the applied target, the applied time, and the applied frequency are the same as those in the section of the "blood circulation promoting external preparation" described above.

A higher blood circulation promoting effect compared to the prior art is obtained as the blood circulation promoting external preparation, blood circulation promoting device, or blood circulation promoting method of the invention is used.

The present inventors have obtained a finding that not only the invention exhibits an excellent blood circulation promoting effect but also carbonic acid hardly escapes into the atmosphere although a vibratory pressure in the ultrasonic region is generated in the blood circulation promoting external preparation and the action of the preparation can be maintained.

This mechanism has not been sufficiently clearly described, but it is believed that this is perhaps because the size of the bubbles of carbonic acid present in carbonated water is significantly small so that the bubbles hardly emerge from the carbonated water in carbonated water produced by utilizing a static mixer or a multi-layer composite hollow fiber membrane.

Carbonic acid usually forms bubbles to escape into the atmosphere when a physical vibration by an ultrasound or the like is applied to carbonated water. In particular, in the case of preparing carbonated water by a chemical technique in which a tablet or the like containing sodium bicarbonate is introduced into water, the bubbles of carbonic acid have a relatively large diameter and are likely to rise up in the carbonated water by buoyancy and to be discharged from the carbonated water when a physical vibration is applied to the carbonated water. In addition, the bubbles are hardly joined with one another when the diameter of the bubbles of carbonic acid is large, thus the buoyancy is further increased, and the bubbles are more easily discharged from the carbonated water.

On the other hand, the bubbles of carbonic acid are small and buoyancy is small in the carbonated water produced by utilizing a static mixer or a multi-layer composite hollow fiber membrane, and thus the bubbles are likely to remain in the carbonated water although a physical vibration is applied to the carbonated water. By such a mechanism, those which contain carbonated water produced by utilizing a static mixer or a multi-layer composite hollow fiber membrane among the blood circulation promoting external preparations of the invention are likely to maintain a high concentration of carbonic acid in water although a vibratory pressure in the ultrasonic region is generated.

Consequently, the present specification has disclosed that the invention exhibits an excellent blood circulation promoting effect as the present inventors have found out that both the blood circulation promotion by carbonated water and the ultrasonic irradiation can be achieved.

### EXAMPLES

### (Example 1)

### <Blood circulation promoting device>

The blood circulation promoting external preparation containing carbonated water of the invention was produced by the blood circulation promoting device of the invention illustrated in Fig. 1.

Specifically, the ultrasound generating nozzle disclosed in JP 2008-206679 A was used for the mechanism to generate a vibratory pressure in the ultrasonic region and the mechanism to generate a vortex in the blood circulation promoting external preparation containing carbonated water.

A heater for thermal insulation "ASD0187-2A" (manufactured by AISEI DENNETUS co., ltd.) was used for the mechanism to heat or keep warm the blood circulation promoting external preparation.

A mixer type dissolving element "GPS-T010" (manufactured by MITSUBISHI RAYON CLEANSUI CO., LTD.) was used for the mechanism to dissolve carbonic acid in water.

### <Evaluation on blood circulation promoting effect>

Two test subjects (test subject 1: 58 year old male and test subject 2: 38 year old male) were subjected to the treatment to be described below to evaluate the blood circulation promoting action.

The evaluation on the blood circulation promoting action was conducted by measuring the blood flow rate (ml/min/100 g) at the treated site using a rheometer "Laser Doppler ALF21R" (manufactured by ADVANCE Co., Ltd.). In addition, the blood flow rate was evaluated as an average value within the measured time.

### <Blood circulation promoting treatment>

The blood circulation promoting external preparation of the invention was prepared by heating fresh water and keeping it warm and then dissolving carbonic acid in the fresh water by the blood circulation promoting device described above under the following conditions. From the hand to the middle of the forearm of each test subject was immersed in the blood circulation promoting external preparation and the immersed site was treated for 20 minutes by an ultrasonic vibratory pressure and a vortex.
Concentration of carbonic acid in blood circulation promoting external preparation: 1000 ppm
Temperature of warm water: 38°C
Ultrasonic pressure (vibratory pressure): 125 dB (in acoustic region of 16 kHz)
Pressure of vortex: 190 kPa

### (Comparative Example 1)

The treatment to immerse from the hand to the middle of the forearm of each test subject in warm water was only conducted.

### (Comparative Example 2)

The treatment was conducted in the same manner as in Example 1 except that carbonic acid was not dissolved in water.

### (Comparative Example 3)

The treatment was conducted in the same manner as in Example 1 except that the treatment with an ultrasound and a vortex was not conducted.

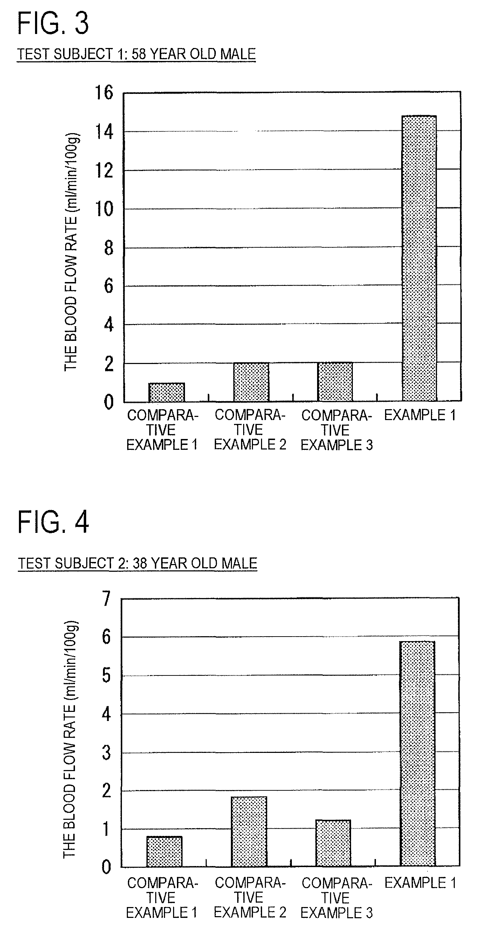

The blood circulation promoting action was evaluated after the treatment according to Example 1 and Comparative Examples 1 to 3 described above. The evaluation results of the blood circulation promoting action on each test subject are illustrated in Figs. 3 and 4, respectively.

From these results, it can be seen that the blood flow rate has synergistically increased in all of the test subjects when all of the treatments with a vibratory pressure in the ultrasonic region, a vortex, and the thermal insulation are combined with the treatment with carbonated water.

### (Examples 2 and 3 and Comparative Examples 4 to 7)

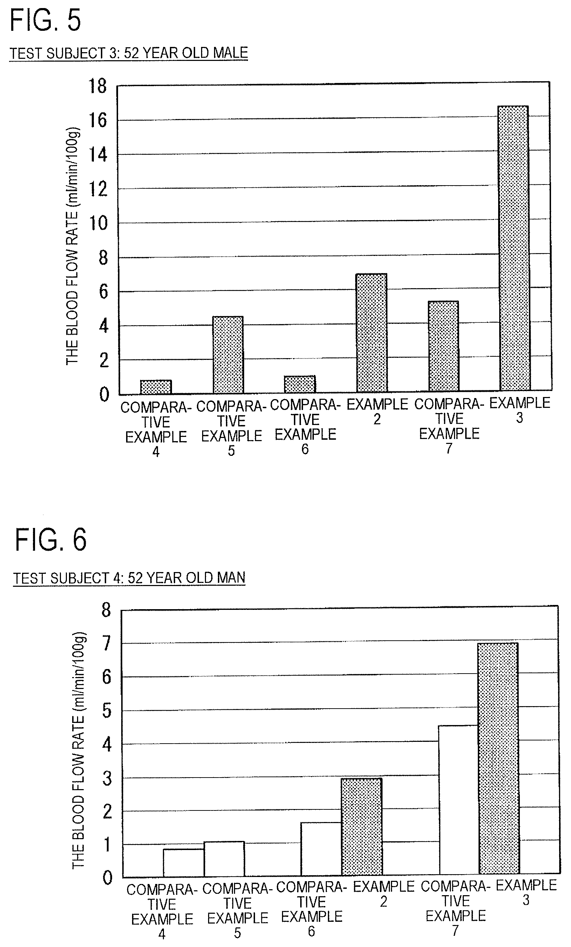

In Examples 2 and 3 and Comparative Examples 4 to 7, two test subjects (test subject 3: 52 year old male and test subject 4: 52 year old man) were subjected to the treatment in the same manner as in Examples 1 above except that the treatment was conducted under the conditions presented in the following Table 1 to evaluate the blood circulation promoting action. The evaluation results are illustrated in Fig. 5.

**[Table 1]**

| | Comparative Example 4 | Comparative Example 5 | Comparative Example 6 | Example 2 | Comparative Example 7 | Example 3 |
|---|---|---|---|---|---|---|
| Concentration of carbonic acid in blood circulation promoting external preparation (ppm) | 1 | 1000 | 1 | 1000 | 1 | 1000 |
| Temperature of water (°C) | 34 | | | | | |
| Ultrasonic vibratory pressure (Hz) | 0 | | 1M | | 3M | |
| Pressure of vortex flow (kPa) | 0 | | | | | |

As a result, an increase in blood flow rate in Examples 2 and 3 in which ultrasonic irradiation was conducted in carbonated water at 34°C having a concentration of carbonic acid of 1000 ppm was higher compared to that in Comparative Examples 4 to 7 in which the concentration of carbonic acid was 1 ppm (fresh water) or the ultrasonic irradiation was not conducted.

### (Examples 4 and 5 and Comparative Examples 8 to 11)

In Examples 4 and 5 and Comparative Examples 8 to 11, the treatment was conducted in the same manner as in Examples 1 above except that the treatment was conducted under the conditions presented in the following Table 2 to evaluate the blood circulation promoting action. The evaluation results are illustrated in Fig. 6.

**[Table 2]**

| | Comparative Example 8 | Comparative Example 9 | Comparative Example 10 | Example 4 | Comparative Example 11 | Example 5 |
|---|---|---|---|---|---|---|
| Concentration of carbonic acid in blood circulation promoting external preparation (ppm) | 1 | | 500 | | 1000 | |
| Temperature of water (°C) | 34 | | | | | |
| Ultrasonic vibratory pressure (Hz) | 0 | 1M | 0 | 1M | 0 | 1M |
| Pressure of vortex flow (kPa) | 0 | | | | | |

As a result, a synergistic effect was obtained in Example 4 in which the blood circulation promoting external preparation having a concentration of carbonic acid of 500 ppm and an ultrasound having a vibratory pressure of 1 MHz were concurrently used as compared to those in Comparative Example 9 in which only an ultrasound was used and Comparative Example 10 in which only the blood circulation promoting external preparation was used.

In addition, a synergistic effect was also obtained in Example 5 in which the blood circulation promoting external preparation having a concentration of carbonic acid 1000 ppm and an ultrasound having a vibratory pressure of 1 MHz were concurrently used as compared to those in Comparative Example 9 in which only an ultrasound was used and Comparative Example 11 in which only carbonated water was used, and the effect was also much higher than that in Example 4.

### (Example 6)

### <Blood circulation promoting device>

The blood circulation promoting external preparation containing carbonated water of the invention was produced by the blood circulation promoting device of the invention illustrated in Fig. 2.

Specifically, the US-750 manufactured by Ito Co., Ltd. was used as the ultrasonic vibratory pressure generating unit 20.

The same ones as those in Example 1 were used for the mechanism to heat or keep warm the blood circulation promoting external preparation and the mechanism to dissolve carbonic acid in water, respectively.

### <Blood circulation promoting treatment>

Two test subjects (test subject 5: 47 year old male and test subject 6: 46 year old male) were subjected to the following treatment to evaluate the blood circulation promoting action by the same method as in Example 1.

The blood circulation promoting external preparation of the invention was prepared by heating fresh water and keeping it warm and then dissolving carbonic acid in the fresh water by the blood circulation promoting device described above under the following conditions. The treated site of the test subject was irradiated with an ultrasound for 20 minutes while continuously applying the blood circulation promoting external preparation to from the wrist to the back of the hand of each test subject using the shower head 16.
Concentration of carbonic acid in blood circulation promoting external preparation: 1000 ppm
Temperature of warm water: 37°C
Shortest distance from tip of transducer 21 of device 20 to target site: 1 cm

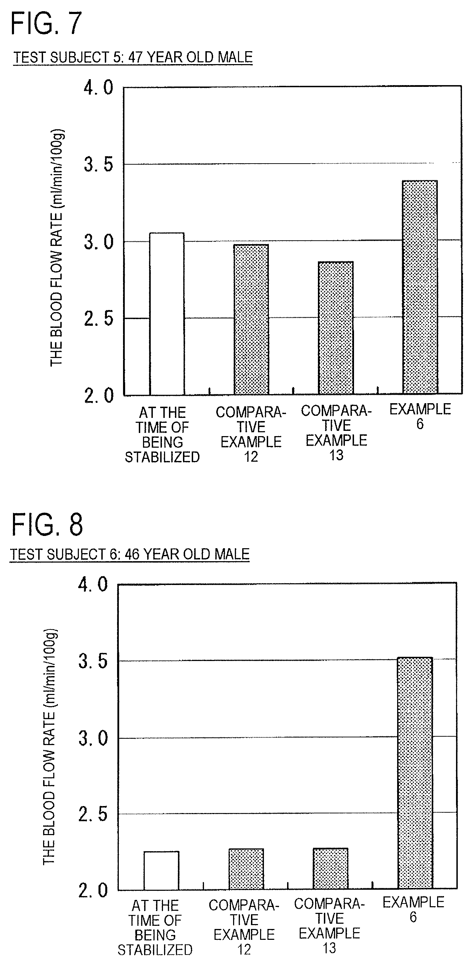

The evaluation results for each test subject are illustrated in Figs. 7 and 8 together with the evaluation results at the time of being stabilized.

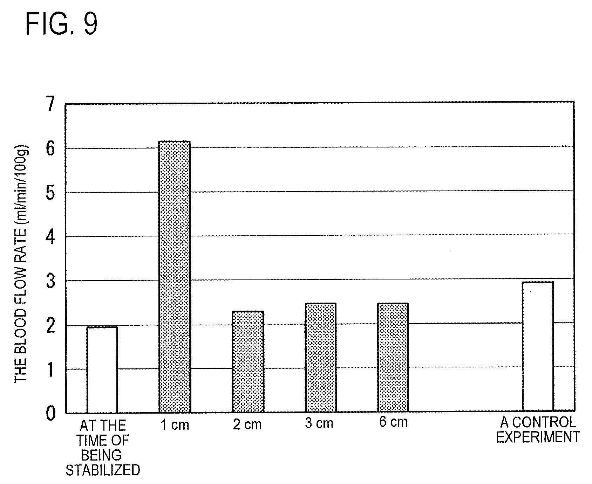

In addition, the shortest distance from the tip of the transducer 21 of the blood circulation promoting device to the target site was changed from 1 cm to 2 cm, 3 cm, and 6 cm in this order for the test subject 5 to evaluate the blood circulation promoting effect. In addition, thereafter, the blood circulation promoting effect when an ultrasound is irradiated under the conditions described above using the fresh water heated to 37°C instead of the carbonated water was evaluated as a control experiment. The results arc illustrated in Fig. 9.

### (Comparative Example 12)

The treatment was conducted in the same manner as in Example 6 except that the ultrasonic irradiation was not conducted. The results are illustrated in Figs. 7 and 8.

### (Comparative Example 13)

The treatment was conducted in the same manner as in Example 6 except that carbonated water was not used. The results are illustrated in Figs. 7 and 8.

As can be seen from the results illustrated in Figs. 7 and 8, a great blood circulation promoting effect is obtained by the combination of carbonated water and ultrasonic irradiation, but a blood circulation promoting effect is not almost confirmed by the treatment with carbonated water alone or ultrasonic irradiation alone.

In addition, from Fig. 9, it can be seen that the blood circulation promoting effect according to the invention is affected by the distance to the subject at the time of ultrasonic irradiation.

### EXPLANATIONS OF LETTERS OR NUMERALS

1 Bathtub
2 and 12 Heating unit
3 Circulation pipe
4 and 14 Pump
5 and 15 Gas dissolving unit
6 Discharge nozzle
7 and 17 Carbonic acid gas pipe
8 and 18 Water inlet
9 and 19 Bomb
10 Carbonated water producing unit
11 Container
13 Pipe
16 Spray or shower head
20 Ultrasonic vibratory pressure generating unit
21 Transducer
22 Cord
23 Ultrasonic vibratory pressure generating device body
31 and 32 Blood circulation promoting device and
40 Target site

## Claims

1. A blood circulation promoting external preparation comprising carbonated water, wherein
the blood circulation promoting external preparation is applied to a target site of a living body together with ultrasonic irradiation.

2. The blood circulation promoting external preparation according to claim 1, wherein a concentration of carbonic acid dissolved in the blood circulation promoting external preparation is 100 ppm or more.

3. The blood circulation promoting external preparation according to claim 1 or 2, wherein the application of the blood circulation promoting external preparation to a target site is carried out by immersion of a target site in the blood circulation promoting external preparation, coating of the blood circulation promoting external preparation on a target site, or continuous or intermittent supply of the blood circulation promoting external preparation to a target site.

4. The blood circulation promoting external preparation according to any one of claims 1 to 3, wherein the ultrasonic irradiation has a step of generating a vibratory pressure in an ultrasonic region in a blood circulation promoting external preparation or with respect to a target site.

5. The blood circulation promoting external preparation according to any one of claims 1 to 4, wherein the carbonated water is prepared at the time of use.

6. The blood circulation promoting external preparation according to any one of claims 1 to 5, wherein the blood circulation promoting external preparation is kept warm at from 30 to 50°C at the time of use.

7. The blood circulation promoting external preparation according to any one of claims 1 to 6, wherein the number of vibrations in an ultrasonic region in the ultrasonic irradiation is from 16 kHz to 10 MHz.

8. The blood circulation promoting external preparation according to any one of claims 1 to 7, wherein the blood circulation promoting external preparation is further used in a state in which a vortex is generated.

9. The blood circulation promoting external preparation according to any one of claims 1 to 8, wherein the blood circulation promoting external preparation is for treatment or prevention of a disease, treatment of an external injury, or beauty.

10. The blood circulation promoting external preparation according to claim 9, wherein the disease is selected from the group consisting of a cardiovascular disease, a bone disease, a skin disease, and an infectious ulcer.

11. A blood circulation promoting device comprising:
an external preparation applying mechanism to apply a blood circulation promoting external preparation containing carbonated water to a target site; and
an ultrasound irradiating mechanism to generate a vibratory pressure in an ultrasonic region and to irradiate the target site with the vibratory pressure.

12. The blood circulation promoting device according to claim 11, wherein a concentration of carbonic acid dissolved in the blood circulation promoting external preparation is 100 ppm or more.

13. The blood circulation promoting device according to claim 11 or 12, wherein the external preparation applying mechanism is constituted so that the application of the blood circulation promoting external preparation to a target site is carried out by immersion of a target site in the blood circulation promoting external preparation, coating of the blood circulation promoting external preparation on a target site, or continuous or intermittent supply of the blood circulation promoting external preparation to a target site.

14. The blood circulation promoting device according to any one of claims 11 to 13, wherein the ultrasound irradiating mechanism has a device to generate a vibratory pressure in an ultrasonic region in the blood circulation promoting external preparation or with respect to a target site.

15. The blood circulation promoting device according to any one of claims 11 to 14, further comprising a mechanism to dissolve carbonic acid in water.

16. The blood circulation promoting device according to any one of claims 11 to 15, further comprising a mechanism to control a temperature of the blood circulation promoting external preparation.

17. The blood circulation promoting device according to any one of claims 11 to 16, further comprising a mechanism to generate a vortex in the blood circulation promoting external preparation.

18. The blood circulation promoting device according to any one of claims 11 to 17, wherein the blood circulation promoting device is for treatment or prevention of a disease, treatment of an external injury, or beauty.

19. The blood circulation promoting device according to claim 18, wherein the disease is selected from the group consisting of a cardiovascular disease, a bone disease, a skin disease, and an infectious ulcer.

20. A blood circulation promoting method comprising,
a step of applying the blood circulation promoting external preparation according to any one of claims 1 to 10 to a target site as well as generating a vibratory pressure in an ultrasonic region and irradiating the target site with the vibratory pressure.

21. The blood circulation promoting method according to claim 20, wherein the blood circulation promoting method is for treatment or prevention of a disease, treatment of an external injury, or beauty.

22. The blood circulation promoting method according to claim 21, wherein the disease is selected from the group consisting of a cardiovascular disease, a bone disease, a skin disease, and an infectious ulcer.

23. The blood circulation promoting method according to any one of claims 20 to 22, wherein the blood circulation promoting external preparation is applied to the target site at a temperature of from 30 to 50°C.
